# EUROPEAN PATENT APPLICATION

(11) **EP 1 421 953 A1**
(43) Date of publication of application: **26.05.2004**
(21) Application number: 02762874.2
(22) Date of filing: 27.08.2002
(51) Int. Cl.: A61K 45/06, A61K 31/165, A61K 31/167, A61K 31/17, A61K 31/255, A61K 31/357, A61K 31/404, A61K 31/41, A61K 31/4178, A61K 31/4184, A61K 31/44, A61K 31/4439, A61K 31/496, A61K 31/662, A61P 9/10

(54) **MEDICINAL COMPOSITIONS CONTAINING ANGIOTENSIN II RECEPTOR ANTAGONIST**

(30) Priority: 28.08.2001 JP 2001257435
(71) Applicant: Sankyo Company, Limited, Tokyo 103-8426 (JP)
(72) Inventor: SADA, Toshio, c/o SANKYO COMPANY, LIMITED, Tokyo 140-8710 (JP); INABA, Toshimori, c/o SANKYO COMPANY, LIMITED, Tokyo 140-8710 (JP)
(74) Representative: Gibson, Christian John Robert
(86) International application number: PCT/JP2002/008629
(87) International publication number: WO 2003/020315

(57) **Abstract**

The present invention relates to a pharmaceutical composition for administering simultaneously, separately or sequentially an angiotensin II receptor antagonist and an ACAT inhibitor.

## Description

### Technical field of the invention

The present invention relates to a pharmaceutical composition for administering simultaneously, separately or sequentially an angiotensin II receptor antagonist and an acylcoenzyme A: cholesterol acyltransferase (hereinafter refered as ACAT) inhibitor.

The present invention also relates to a method for the prevention or treatment of arteriosclerosis or diseases resulting from arteriosclerosis such as ischemic heart disease, ischemic brain disease, peripheral circulatory failure, and the like, which comprises administering simultaneously, separately or sequentially a pharmacologically effective amount of an angiotensin II receptor antagonist and an ACAT inhibitor to a warm-blooded animal (particularly a human).

### Background of the Invention

The incidence of arteriosclerosis is continuing to increase accompanying the introduction of a Western diet and growing proportion of elderly people in the population. Arteriosclerosis may be the primary causes of ischemic heart diseases (myocardial infarction, unstable angina, and ischemic sudden death), ischemic brain disorders (cerebral infarction, cerebral apoplexy, and so forth), peripheral circulatory failure, and the like. In addition to hyperlipidemia (and particularly hypercholesterolemia), risk factors for arteriosclerosis include hypertension, and abnormalities in carbohydrate metabolism based on insulin resistance. These risk factors are considered to enhance complications of the above etiological diseases and have been proposed as syndrome X [Diabetes, 37, 1595 (1988)]. An innovative development of either prophylactic or therapeutic interventions for these complications is now urgently desired.

### Disclosure of the Invention

The present inventors have eagerly studied a pharmaceutical composition comprising an angiotensin II receptor antagonist and an ACAT inhibitor. As a result, it has been found that the pharmaceutical composition comprising an angiotensin II receptor antagonist and an ACAT inhibitor has a more prominent suppressive action against progression of arteriosclerosis with low toxicity, when both active ingredients are administered in combination (simultaneously, separately or sequentially), rather than when either one is administered alone. Furthermore, the present inventors have found that this combination therapy is useful as a preventive or therapeutic agent (particularly a therapeutic agent) for warm-blooded animals (particularly humans) with diseases resulting from arteriosclerosis such as ischemic heart disease, ischemic brain diseases, and peripheral circulatory failure, and thus have completed the present invention.

The present invention provides a pharmaceutical composition (particularly a pharmaceutical composition for the prevention or treatment of arteriosclerosis or arteriosclerosis-derived diseases such as ischemic heart disease, ischemic brain disease, peripheral circulatory failure, and the like) for administering simultaneously, separately or sequentially an angiotensin II receptor antagonist and an ACAT inhibitor.

The present invention also provides a method for the prevention or treatment of arteriosclerosis or arteriosclerosis-derived diseases such as ischemic heart disease, ischemic brain disease, peripheral circulatory failure, and the like, which comprises administering simultaneously, separately or sequentially a pharmacologically effective amount of an angiotensin II receptor antagonist and an ACAT inhibitor to a warm-blooded animal (particularly a human).

The active ingredients of the pharmaceutical composition of the present invention are an angiotensin II receptor antagonist and an ACAT inhibitor.

The "angiotensin II receptor antagonist" that is one of the active ingredients of the pharmaceutical composition of the present invention blocks vasoconstrictive action of angiotensin II by binding to angiotensin II receptors located on the cell surface membrane, and was originally developed as an antihypertensive agent.

Such an angiotensin II receptor antagonist includes, for example, a biphenyl tetrazole compound or biphenylcarboxylic acid derivative such as:
a compound having the general formula (I) or a pharmacologically acceptable salt thereof disclosed in the Japanese Patent Publication (Kokai) Sho 63-23868 (USP 5,138,069) {preferably losartan, of which the chemical name is 2-butyl-4-chloro-1-(2'-(1H-tetrazol-5-yl)biphenyl-4-ylmethyl]-1H-imidazol-5-methanol, and in the present invention losartan includes pharmacologically acceptable salts of losartan (losartan potassium salt or the like).};
a compound having the general formula (I) or a pharmacologically acceptable salt thereof disclosed in the Japanese Patent Publication (Kohyo) Hei 4-506222 (WO 91/14679) {preferably irbesartan, of which the chemical name is 2-N-butyl-4-spirocyclopentane-1-[2'-(1H-tetrazol-5-yl)biphenyl-4-ylmethyl]-2-imidazoline-5-one, and in the present invention irbesartan includes pharmacologically acceptable salts of irbesartan.};
a compound having the general formula (I), an ester thereof, or a pharmacologically acceptable salt thereof disclosed in the Japanese Patent Publication (Kokai) Hei 4-235149 (EP 433983) {preferably valsartan, of which the chemical name is (S)-N-valeryl-N-[2'-(1H-tetrazol-5-yl)biphenyl-4-ylmethyl]valine, and in the present invention valsartan includes pharmacologically acceptable esters and pharmacologically acceptable salts of valsartan.};
a carboxylic acid derivative having the general formula (I), an ester thereof, or a pharmacologically acceptable salt thereof disclosed in the Japanese Patent Publication (Kokai) Hei 4-364171 (USP 5,196,444) {preferably candesartan, of which the chemical name is 1-(cyclohexyloxycarbonyloxy)ethyl 2-ethoxy-1-[2'-(1H-tetrazol-5-yl)biphenyl-4-ylmethyl]-1H-benzimidazole-7-carboxylate, and in the present invention candesartan includes carboxylic acid derivatives thereof, pharmacologically acceptable esters of the carboxylic acid derivatives (TCV-116 or the like), and pharmacologically acceptable salts thereof.};
a carboxylic acid derivative having the general formula (I), an ester thereof, or a pharmacologically acceptable salt thereof disclosed in the Japanese Patent Publication (Kokai) Hei 5-78328 (USP 5,616,599) {preferably olmesartan, of which the chemical name is (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate, and in the present invention olmesartan includes carboxylic acid derivatives thereof, pharmacologically acceptable esters of the carboxylic acid derivatives (CS-866 or the like), and pharmacologically acceptable salts thereof.}; and
a compound having the general formula (I), an ester thereof, or a pharmacologically acceptable salt thereof disclosed in the Japanese Patent Publication (Kokai) Hei 4-346978 (USP 5,591,762, EP 502,314) {preferably telmisartan, of which the chemical name is 4'-[[2-n-propyl-4-methyl-6-(1-methylbenzimidazol-2-yl)-benzimidazol-1-yl]methyl]biphenyl-2-carboxylate, and in the present invention telmisartan includes carboxylic acid derivatives thereof, pharmacologically acceptable esters of the carboxylic acid derivatives, and pharmacologically acceptable salts thereof.}.

The angiotensin II receptor antagonist preferably is losartan, irbesartan, valsartan, candesartan, olmesartan, or telmisartan; more preferred is losartan or olmesartan; and most preferred is olmesartan.

The planar chemical structures of the preferred angiotensin II receptor antagonists are shown below.

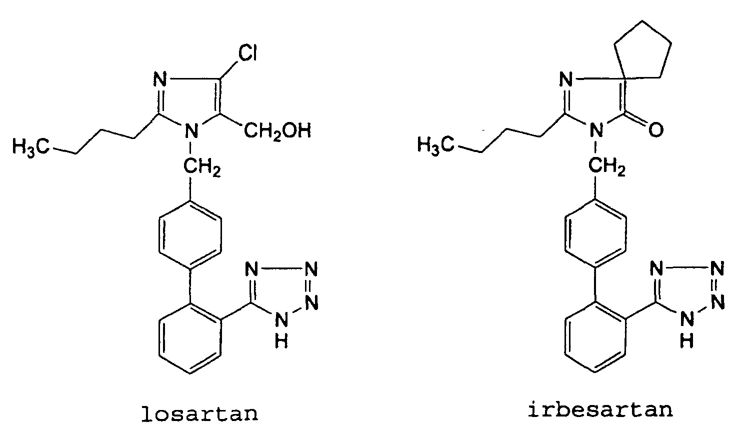

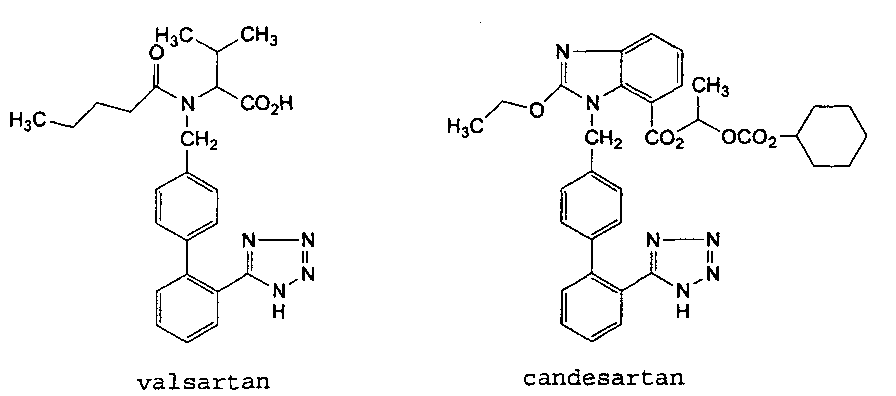

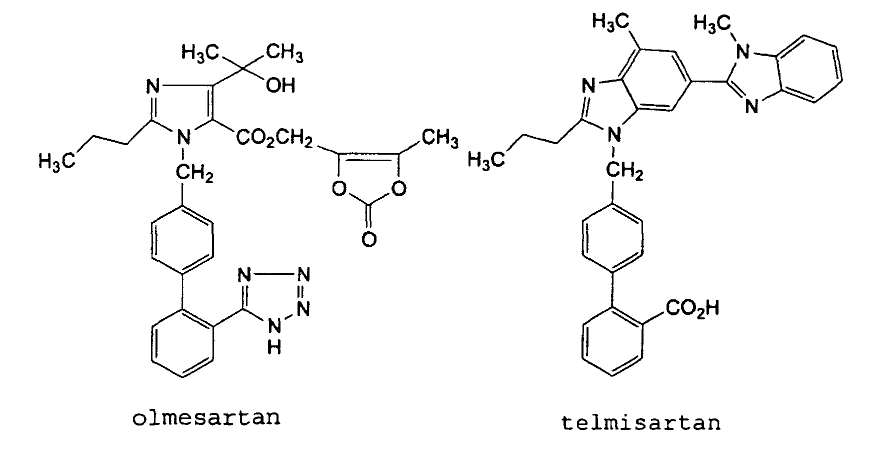

The "ACAT inhibitor" that is one of the active ingredients of the pharmaceutical composition of the present invention, is an agent that inhibits acylcoenzyme A: cholesterol acyltransferase (ACAT), and not only decreases cholesterol but also acts directly on arteriosclerotic lesions of blood vessel walls and then inhibits the formation of macrophage foam cells (inhibits accumulation of cholesterol in the cells). The inhibitor was originally designed as an agent for preventing or treating hyperlipidemia or arteriosclerosis.

Such an ACAT inhibitor includes, for example:
a compound having the general formula (I) disclosed in WO 92/09561 [preferably FR-129169, of which the chemical name is N-(1,2-diphenylethyl)-2-(2-octyloxyphenyl)acetamide];
a compound having the general formula (I) or a pharmacologically acceptable salt thereof disclosed in the Japanese Patent Publication (Kohyo) Hei 8-510256 (WO 94/26702, USP 5,491,172) {preferably CI-1011, of which the chemical name is 2,6-diisopropylphenyl-N-[(2,4,6-triisopropylphenyl)acetyl]sulfamate, and in the present invention CI-1011 includes pharmacologically acceptable salts of CI-1011.};
a compound having the general formula (I) or a pharmacologically acceptable salt thereof disclosed in EP 421441 (USP 5,120,738) {preferably F-1394, of which the chemical name is (1S,2S)-2-[3-(2,2-dimethylpropyl)-3-nonylureido]cyclohexan-1-yl 3-[(4R)-N-(2,2,5,5-tetramethyl-1,3-dioxane-4-carbonyl)amino]propionate, and in the present invention F-1394 includes pharmacologically acceptable salts of F-1394.);
a compound or a pharmacologically acceptable salt thereof disclosed in the Japanese Patent Publication (Kohyo) 2000-500771 (WO 97/19918, USP 5,990,173) [preferably F-12511, of which the chemical name is (S)-2',3',5'-trimethyl-4'-hydroxy-α-dodecylthio-α-phenylacetanilide, and in the present invention F-12511 includes pharmacologically acceptable salts of F-12511.];
a compound having the general formula (I) or a pharmacologically acceptable salt thereof disclosed in the Japanese Patent Publication (Kokai) Hei 10-195037 (EP 790240, USP 5,849,732) [preferably T-2591, of which the chemical name is 1-(3-t-butyl-2-hydroxy-5-methoxyphenyl)-3-(2-cyclohexylethyl)-3-(4-dimethylaminophenyl)urea, and in the present invention T-2591 includes pharmacologically acceptable salts (hydrochloride or the like) of T-2591.];
a compound having the general formula (I) or a pharmacologically acceptable salt thereof disclosed in WO 96/26948 {preferably FCE-28654, of which the chemical name is 1-(2,6-diisopropylphenyl)-3-[(4R,5R)-4,5-dimethyl-2-(4-phosphonophenyl)-1,3-dioxolan-2-ylmethyl]urea, and in the present invention FCE-28654 includes pharmacologically acceptable salts (sodium salt or the like) of FCE-28654.};
a compound having the general formula (I) or a pharmacologically acceptable salt thereof disclosed in the specification of WO 98/54153 (EP 987254) {preferably K-10085, of which the chemical name is N-[2,4-bis(methylthio)-6-methyl-3-pyridyl]-2-[4-[2-(oxazolo[4,5-b]pyridin-2-ylthio)ethyl]piperazin-1-yl]acetamide, and in the present invention K-10085 includes pharmacologically acceptable salts and solvates of K-10085.};
a compound having the general formula (I) disclosed in WO 92/09572 (EP 559898, USP 5,475,130) [preferably HL-004, of which the chemical name is N-(2,6-diisopropylphenyl)-2-tetradecylthioacetamide.];
a compound having the general formula (I) or a pharmacologically acceptable salt thereof disclosed in the Japanese Patent Publication (Kokai) Hei 7-82232 (EP 718281) {preferably NTE-122, of which the chemical name is trans-1,4-bis[1-cyclohexyl-3-(4-dimethylaminophenyl)ureidomethylJcyclohexane, and in the present invention NTE-122 includes pharmacologically acceptable salts of NTE-122.};
a compound or a pharmacologically acceptable salt thereof disclosed in the Japanese Patent Publication (Kohyo) Hei 10-510512 (WO 96/10559) {preferably FR-186054, of which the chemical name is 1-benzyl-1-[3-(pyrazol-3-yl)benzyl]-3-[2,4-bis(methylthio)-6-methylpyridin-3-yl]urea, and in the present invention FR-186054 includes pharmacologically acceptable salts of FR-186054.);
a compound having the general formula (I) or a pharmacologically acceptable salt thereof disclosed in WO 96/09287 (EP 0782986, USP 5,990,150) [preferably N-(1-pentyl-4,6-dimethylindolin-7-yl)-2,2-dimethylpropaneamide, and in the present invention includes pharmacologically acceptable salts thereof (hydrochloride or the like).]; and
a compound having the general formula (I) or a pharmacologically acceptable salt thereof disclosed in WO 97/12860 (EP 0866059, USP 6,063,806) [preferably N-(1-octyl-5-carboxymethyl-4,6-dimethylindolin-7-yl)-2,2-dimethylpropaneamide, and in the present invention includes pharmacologically acceptable salts thereof (hydrochloride, sulfate, or the like).].

The ACAT inhibitor preferably is a compound selected from the group consisting of FR-129169, CI-1011, F-1394, F-12511, T-2591, FCE-28654, K-10085, HL-004, NTE-122, FR-186054, N-(1-octyl-5-carboxymethyl-4,6-dimethylindolin-7-yl)-2,2-dimethylpropaneamide (hereinafter referred as compound A), and N-(1-pentyl-4,6-dimethylindolin-7-yl)-2,2-dimethylpropaneamide (hereinafter referred as compound B), or a pharmacologically acceptable salt thereof.

The ACAT inhibitor more preferably is a compound selected from the group consisting of CI-1011, F-12511, N-(1-octyl-5-carboxymethyl-4,6-dimethylindolin-7-yl)-2,2-dimethylpropaneamide (compound A), and N-(1-pentyl-4,6-dimethylindolin-7-yl)-2,2-dimethylpropaneamide (compound B), or a pharmacologically acceptable salt thereof; most preferred is N-(1-octyl-5-carboxymethyl-4,6-dimethylindolin-7-yl)-2,2-dimethylpropaneamide (compound A) or a pharmacologically acceptable salt thereof.

The planar chemical structures of the preferred ACAT inhibitors are shown below.

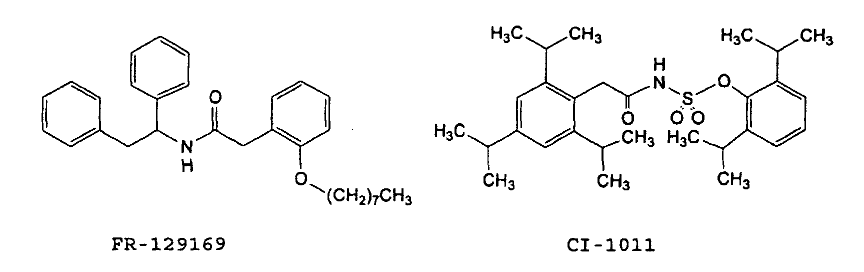

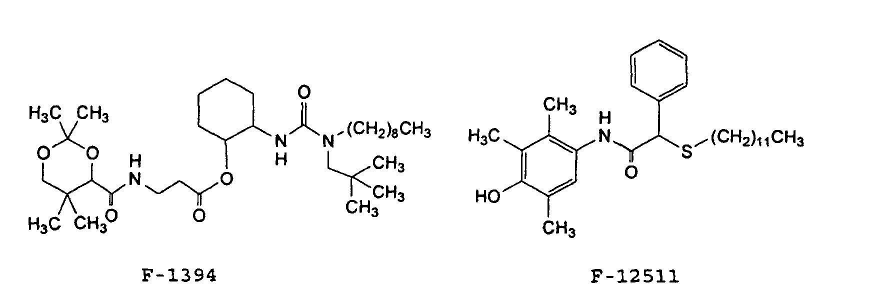

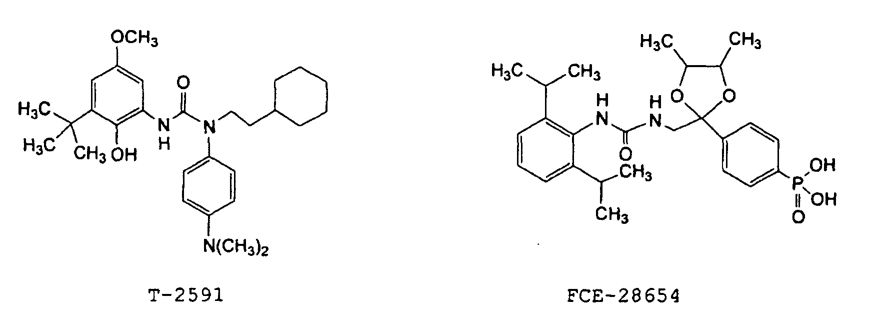

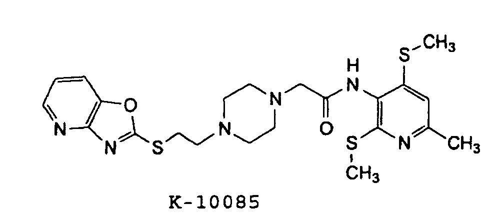

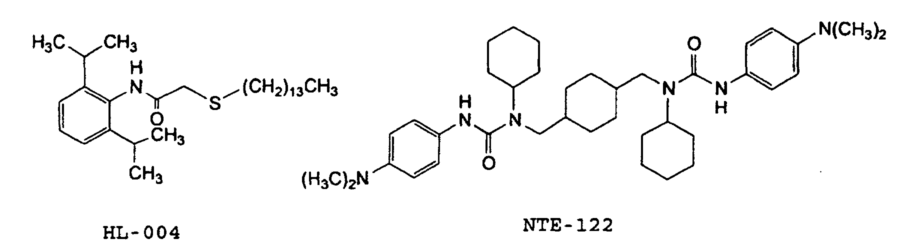

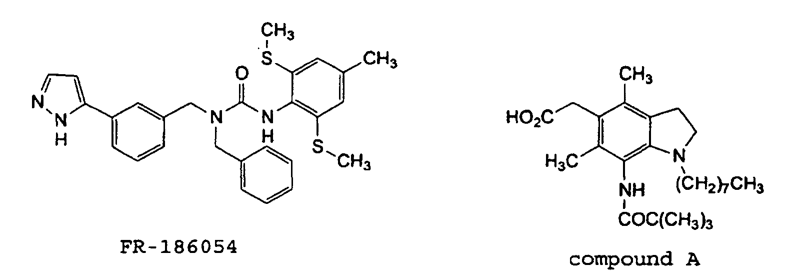

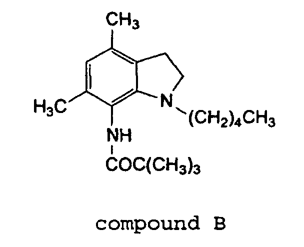

When the angiotensin II receptor antagonist and the ACAT inhibitor, the active ingredients of the pharmaceutical composition of the present invention, have a basic group such as an amino group, the salts of these compounds can be prepared by reacting the compounds with an acid, and when the angiotensin II receptor antagonist and the ACAT inhibitor have an acidic group such as a carboxyl group, the salts of these compounds can be prepared by reacting the compounds with a base. Such salts are included in the present invention.

The preferred salt, when the original antagonist and inhibitor have a basic group, includes an inorganic acid salt, for example, a hydrohalogenate such as hydrofluoride, hydrochloride, hydrobromide, or hydroiodide, nitrate, perchlorate, sulfate, phosphate or the like; an organic acid salt, for example, a lower alkanesulfonate such as methanesulfonate, trifluoromethanesulfonate, or ethanesulfonate, an arylsulfonate such as benzenesulfonate or p-toluenesulfonate, acetate, malate, fumarate, succinate, citrate, ascorbate, tartarate, oxalate, maleate, or the like; or an amino acid salt such as glycine salt, lysine salt, arginine salt, ornitine salt, glutamate, or aspartic acid salt.

The preferred salt, when the original antagonist and inhibitor have an acidic group, includes a metal salt, for example, an alkali metal salt such as sodium salt, potassium salt, or lithium salt, an alkaline earth metal salt such as calcium salt or magnesium salt, aluminum salt, iron salt, or the like; an amine salt, for example, an inorganic salt such as ammonium salt, an organic acid salt such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt, tris(hydroxymethyl)aminomethane salt, or the like; or an amino acid salt such as glycine salt, lysine salt, arginine salt, ornitine salt, glutamic acid salt, or aspartic acid salt.

When the angiotensin II receptor antagonist and the ACAT inhibitor, the active ingredients of the pharmaceutical composition of the present invention, have asymmetric carbons in their structures, these compounds can exist as various stereoisomers due to such asymmetric carbons. In the present invention these compounds are represented as a single chemical formula individually. The present invention encompasses both individual stereoisomers and mixtures of two or more stereoisomers in any ratio.

When the angiotensin II receptor antagonist and the ACAT inhibitor, the active ingredients of the pharmaceutical composition of the present invention, are allowed to stand in contact with the atmosphere or to recrystallize, they may absorb water or water may attach on them to form a hydrate. The present invention encompasses such hydrates.

The pharmaceutical composition of the present invention is a pharmaceutical composition for administering simultaneously, separately or sequentially an angiotensin II receptor antagonist and an ACAT inhibitor.

In the present invention a dosage form for administration of the two agents "simultaneously" is not particularly limited provided that the two agents can be administered almost at the same time. The preferred dosage form is a single unit dosage form containing the two agents.

In the present invention a dosage form for administration of the two agents "separately or sequentially " is not particularly limited provided that the two agents can be separately administered wherein there is a time interval between the first administration, of one agent, and the second administration, of the other agent. For example, after a pre-determined time from the first administration of the ACAT inhibitor, the angiotensin II receptor antagonist is administered, or after pre-determined time from the first administration of the angiotensin II receptor antagonist, the ACAT inhibitor is administered.

The pharmaceutical composition of the present invention relates to
(1) a pharmaceutical composition for administering simultaneously, separately or sequentially an angiotensin II receptor antagonist and an ACAT inhibitor.
   Preferred pharmaceutical compositions include
(2) a pharmaceutical composition according to (1) wherein the angiotensin II receptor antagonist is losartan, irbesartan, valsartan, candesartan, olmesartan, or telmisartan;
(3) a pharmaceutical composition according to (1) wherein the angiotensin II receptor antagonist is losartan or olmesartan;
(4) a pharmaceutical composition according to (1) wherein the angiotensin II receptor antagonist is olmesartan;
(5) a pharmaceutical composition according to any one of (1) to (4) wherein the ACAT inhibitor is FR-129169, CI-1011, F-1394, F-12511, T-2591, FCE-28654, K-10085, HL-004, NTE-122, FR-186054, N-(1-octyl-5-carboxymethyl-4,6-dimethylindolin-7-yl)-2,2-dimethylpropaneamide, or N-(1-pentyl-4,6-dimethylindolin-7-yl)-2,2-dimethylpropaneamide or a pharmacologically acceptable salt thereof;
(6) a pharmaceutical composition according to any one of (1) to (4) wherein the ACAT inhibitor is N-(1-octyl-5-carboxymethyl-4,6-dimethylindolin-7-yl)-2,2-dimethylpropaneamide or a pharmacologically acceptable salt thereof.
   The preferred pharmaceutical compositions also include pharmaceutical compositions which are an optional combination of an angiotensin II receptor antagonist, an active ingredient of the pharmaceutical composition, selected from (2) to (4), and an ACAT inhibitor, an active ingredient of the pharmaceutical composition, selected from (5) or (6). Examples of such a combination are shown below:
(7) a pharmaceutical composition according to (1) wherein the angiotensin II receptor antagonist, an active ingredient of the pharmaceutical composition, is losartan, irbesartan, valsartan, candesartan, olmesartan, or telmisartan, and the ACAT inhibitor, an active ingredient of the pharmaceutical composition, is FR-129169, CI-1011, F-1394, F-12511, T-2591, FCE-28654, K-10085, HL-004, NTE-122, FR-186054, N-(1-octyl-5-carboxymethyl-4,6-dimethylindolin-7-yl)-2,2-dimethylpropaneamide, or N-(1-pentyl-4,6-dimethylindolin-7-yl)-2,2-dimethylpropaneamide or a pharmacologically acceptable salt thereof;
(8) a pharmaceutical composition according to (1) wherein the angiotensin II receptor antagonist, an active ingredient of the pharmaceutical composition, is losartan, or olmesartan, and the ACAT inhibitor, an active ingredient of the pharmaceutical composition, is N-(1-octyl-5-carboxymethyl-4,6-dimethylindolin-7-yl)-2,2-dimethylpropaneamide, or a pharmacologically acceptable salt thereof; or
(9) a pharmaceutical composition according to (1) wherein the angiotensin II receptor antagonist, an active ingredient of the pharmaceutical composition, is olmesartan, and the ACAT inhibitor, an active ingredient of the pharmaceutical composition, is N-(1-octyl-5-carboxymethyl-4,6-dimethylindolin-7-yl)-2,2-dimethylpropaneamide, or a pharmacologically acceptable salt thereof.

The angiotensin II receptor antagonists, active ingredients of the pharmaceutical composition of the present invention, can easily be prepared according to procedures described in the specifications of Japanese Patent Publication (Kokai) Sho 61-267541 (EP 188248, USP 4,771,072), WO 99/32478 (USP 5,994,391, USP 6,107,494, USP 2002013476), Japanese Patent Publication (Kokai) Hei 5-310634 (EPP 597107, EP 701991), WO 99/35135, WO 99/64409 (USP 6,221,897), WO 00/47568, WO 00/61568 (USP 6,277,831), and EP 1070703.

The ACAT inhibitors, active ingredients of the pharmaceutical composition of the present invention, can easily be prepared according to procedures described in the specifications of WO 92/09561, Japanese Patent Publication (Kohyo) Hei 8-510256 (WO 94/26702, USP 5,491,172), EP 421441 (USP 5,120,738), Japanese Patent Publication (Kohyo) 2000-50771 (WO 97/19918, USP 5,990,173), Japanese Patent Publication (Kokai) Hei 10-195037 (EP 790240, USP 5,849,732), WO 96/26948, WO 98/54153 (EP 987254), WO 92/09572 (EP 559898, USP 5,475,130), Japanese Patent Publication (Kokai) Hei 7-82232 (EP 718281), Japanese Patent Publication (Kohyo) Hei 10-510512 (WO 96/10559), WO 96/09287 (EP 0782986, USP 5,990,150), and WO 97/12860 (EP 0866059, USP 6,063,806).

### Effects of the invention

The pharmaceutical composition for administering simultaneously, separately or sequentially an angiotensin II receptor antagonist and an ACAT inhibitor has a more prominent suppressive action against progression of arteriosclerosis with low toxicity and therefore is useful as a preventive or therapeutic agent (particularly therapeutic agent) for arteriosclerosis or diseases resulting from arteriosclerosis such as ischemic heart disease, ischemic brain diseases or peripheral circulatory failure for warm-blooded animals (particularly humans). Incidentally, the angiotensin II receptor antagonist and the ACAT inhibitor in the present invention exhibit excellent effects when administered in combination rather than when either is administered alone.

### Industrial applicability

As described hereinbefore the angiotensin II receptor antagonist and the ACAT inhibitor, the active ingredients of the pharmaceutical composition of the present invention, can be prepared in the form of two separate administration units or in the form of a single administration unit by physically mixing the two agents.

In cases where the pharmaceutical composition of the present invention is used as a preventive or therapeutic agent for the disorders described above, each of the angiotensin II receptor antagonist and the ACAT inhibitor, the active ingredients of the pharmaceutical composition of the present invention, may be administered orally in formulations such as tablets, capsules, granules, powders, or syrups or administered parenterally in formulations such as injections or suppositories by optionally mixing with a pharmacologically acceptable excipient, diluent, and the like.

Preparations are prepared by conventionally known methods using additive agents such as excipients (for instance, organic excipients including sugar derivatives such as lactose, sucrose, glucose, mannitol, and sorbitol; starch derivatives such as corn starch, potato starch, α-starch, and dextrin; cellulose derivatives such as crystalline cellulose; gum arabic; dextran; pullulan; and inorganic excipients including silicate derivatives such as light anhydrous silicic acid, synthetic aluminum silicate, calcium silicate, and magnesium aluminometasilicate; phosphates such as calcium hydrogenphosphate; carbonates such as calcium carbonate; and sulfates such as calcium sulfate), lubricants (for instance, stearic acid, metal salts of stearic acid such as calcium stearate and magnesium stearate; talc; colloidal silica; waxes such as veegum and spermaceti; boric acid; adipic acid; sulfates such as sodium sulfate; glycol; fumaric acid; sodium benzoate; DL-leucine; sodium fatty acid; laurylsulfates such as sodium lauryl sulfate and magnesium lauryl sulfate; silicates such as silicic anhydride and silicic hydrate; and starch derivatives described above can be listed), binders (for instance, hydroxypropylcellulose, hydroxypropylmethylcellulose, polyvinylpyrrolidone, Macrogol, and similar excipients described above), disintegrants (for instance, cellulose derivatives such as low-substituted hydroxypropylcellulose, carboxymethylcellulose, calcium carboxymethylcellulose, and internal crosslinked-sodium carboxymethylcellulose; chemically modified starch/cellulose derivatives such as carboxymethylstarch, sodium carboxymethylstarch, crosslinked polyvinylpyrrolidone), stabilizers (for instance, para-oxy benzoates such as methyl parahydroxybenzoate and propyl parahydroxybenzoate; alcohols such as chlorobutanol, benzylalcohol, and phenylethylalcohol; benzalkonium chloride; phenols such as phenol and cresol; thimerosal; dehydroacetic acid; and sorbic acid), flavors (for instance, conventionally employed sweeteners, acidifiers, and flavors), diluents, and the like.

The dose and rate of administration of the angiotensin II receptor antagonist and the ACAT inhibitor, the active ingredients of the pharmaceutical composition of the present invention, depend on various factors such as the activities of the pharmaceutical agents and symptoms, age, and body weight of the patients.

The dose varies depending on the symptom, age, etc. of the patients. For example, in the case of oral administration, it is desirable to administer 0.1 mg (preferably 0.5 mg) as a lower limit and 1000 mg (preferably 500 mg) as an upper limit per one time for an adult and one to six times per day depending on the symptoms of the patients.

In the case of intravenous administration, it is desirable to administer 0.01 mg (preferably 0.05 mg) as a lower limit and 100 mg (preferably 50 mg) as an upper limit per one time for an adult and one to six times per day depending on the symptoms of the patients.

Dosing rates of administration of the angiotensin II receptor antagonist and the ACAT inhibitor, the active ingredients of the pharmaceutical composition of the present invention, may also be widely varied. For example, the rates of administration of the angiotensin II receptor antagonist and the ACAT inhibitor are 1:500 to 500:1 as their weight ratios.

### Best mode for carrying out the invention

The present invention will further be exemplified in more detail by the Examples and Formulation examples. However the scope of the present invention is not limited by these Examples.

### Example 1

### Determination of aortic lesioned area

Watanabe heritable hyperlipidemic (WHHL) rabbits aged 16 weeks were divided in 4 groups consisting of 12 or 13 animals each: control group (5% gum arabic solution), olmesartan (0.5 mg/kg) group, compound A (20 mg/kg) group, and combination group of olmesartan (0.5 mg/kg) and compound A (20 mg/kg). These test agents were orally administered for 24 weeks. After the 24^{th} week, the rabbits were sacrificed and their aorta were isolated to determine the aortic lumen area and arteriosclerotic lesion area in the aortic lumen. Then the percentage of arteriosclerotic lesion area in the aortic lumen [arteriosclerotic lesion area in the aortic lumen/the aortic lumen area] × 100 (%)] was calculated. The results are presented in Table 1. In the table, compound A indicates N-(1-octyl-5-carboxymethyl-4,6-dimethylindolin-7-yl)-2,2-dimethylpropaneamide sulfate.

**Table 1**

| group | lesion area in % |
|---|---|
| control group | 59.7 |
| olmesartan group | 49.3 |
| compound A group | 59.0 |
| combination group of | |
| olmesartan and | 46.2 |
| compound A | |

From the above results, it was clearly shown that the arteriosclerotic lesion area in the olmesartan-administered group was reduced compared with that in the control group, while that in the compound A-administered group was little affected. However, the arteriosclerotic lesion area in the combination group of olmesartan and compound A was decreased more potently than those in either single-administration group, indicating that the combination of the two agents exerted excellent suppressive action against progression of arteriosclerosis.

Furthermore, in a similar experiment to that in Example 1, the intima media thickness (rate of lesion area), rate of extracellular matrix area in the lesion region, rate of the smooth muscle cells in the lesion area, rate of infiltrated area of leukocytes in the lesion region, and lipid contents in aorta were determined following Elastica Masson staining and immunohistochemical staining of aortic slice preparations. The results indicate that the combination group of olmesartan and compound A exerted excellent suppressive effects on progression of arteriosclerosis.

### Formulation example 1

### Tablets

| | |
|---|---|
| olmesartan | 50.0 mg |
| compound A | 10.0 mg |
| lactose | 113.0 mg |
| cornstarch | 25.0 mg |
| magnesium stearate | 2.0 mg |
| | 200 mg |

The powders described above are mixed well, and tableted with a tableting machine to prepare a tablet containing 200 mg.

In the above formula, compound A indicates N-(1-octyl-5-carboxymethyl-4,6-dimethylindolin-7-yl)-2,2-dimethylpropaneamide sulfate.

## Claims

1. A pharmaceutical composition for administering simultaneously, separately or sequentially an angiotensin II receptor antagonist and an ACAT inhibitor.

2. A pharmaceutical composition according to claim 1 wherein the angiotensin II receptor antagonist is losartan, irbesartan, valsartan, candesartan, olmesartan, or telmisartan.

3. A pharmaceutical composition according to claim 1 wherein the angiotensin II receptor antagonist is losartan or olmesartan.

4. A pharmaceutical composition according to claim 1 wherein the angiotensin II receptor antagonist is olmesartan.

5. A pharmaceutical composition according to any one of claims 1 to 4 wherein the ACAT inhibitor is FR-129169, CI-1011, F-1394, F-12511, T-2591, FCE-28654, K-10085, HL-004, NTE-122, FR-186054, N-(1-octyl-5-carboxymethyl-4,6-dimethylindolin-7-yl)-2,2-dimethylpropaneamide, or N-(1-pentyl-4,6-dimethylindolin-7-yl)-2,2-dimethylpropaneamide or a pharmacologically acceptable salt thereof.

6. A pharmaceutical composition according to any one of claims 1 to 4 wherein the ACAT inhibitor is N-(1-octyl-5-carboxymethyl-4,6-dimethylindolin-7-yl)-2,2-dimethylpropaneamide or a pharmacologically acceptable salt thereof.

7. A pharmaceutical composition for administering simultaneously, separately or sequentially olmesartan and N-(1-octyl-5-carboxymethyl-4,6-dimethylindolin-7-yl)-2,2-dimethylpropaneamide.

8. A pharmaceutical composition according to any one of claims 1 to 7 for prevention or treatment of arteriosclerosis.

9. A pharmaceutical composition according to any one of claims 1 to 7 for prevention or treatment of ischemic heart disease.
